# EUROPEAN PATENT SPECIFICATION

(11) **EP 2 052 686 B1**
(45) Date of publication and mention of the grant of the patent: **18.08.2010**
(21) Application number: 08018496.3
(22) Date of filing: 22.10.2008
(51) Int. Cl.: A61B 8/00, A61B 8/06

(54) **Ultrasonic diagnostic apparatus and control program thereof**
Diagnostisches Ultraschallgerät und Steuerungsprogramm dafür
Appareil de diagnostic ultrasonique et son programme de contrôle

(30) Priority: 23.10.2007 JP 2007275378
(43) Date of publication of application: 29.04.2009
(73) Proprietor: Kabushiki Kaisha Toshiba, Minato-ku, Tokyo 105-8001 (JP); Toshiba Medical Systems Corporation, Otawara-shi Tochigi 324-8550 (JP)
(72) Inventor: Tetsuya, Yoshida, Otawara-shi Tochigi 324-8550 (JP)
(74) Representative: Kramer - Barske - Schmidtchen

(56) References cited:
- EP-A- 1 354 555
- EP-A- 1 568 323
- EP-A- 1 820 452
- US-A- 6 149 597
- US-A1- 2005 245 828
- US-A1- 2007 167 799
- US-B1- 6 491 633

## Description

The present invention relates to an ultrasonic diagnostic apparatus having a function of clearing away bubbles filling a scan region in a contrast echo method using a contrast medium and to a control program thereof.

An ultrasonic diagnosis is convenient since heart beat or how an embryo moves can be displayed in real time by a simple operation, i.e., just applying an ultrasonic probe from a surface of a body, an examination can be repeatedly carried out because of high safety, a scale of a system is smaller than those of other diagnostic devices such as X-ray, CT, and MRI devices, and an examination at bed side can be readily performed. Further, a small ultrasonic diagnostic apparatus that can be carried in one hand has been developed although its size varies depending on types of functions provided thereto, and an ultrasonic diagnosis does not have an influence of exposure as different from an X-ray and can be used in a maternity hospital or a home-care setting.

In recent years, an intravenous dosage type ultrasonic contrast medium has been commercialized, and a "contrast echo method" has been carried out. This technique is intended to inject an ultrasonic contrast medium from a vein in, e.g., an examination of a heart or a liver to increase an intensity of a signal from flowing blood, thereby evaluating a moving state of a blood flow. Many contrast mediums have micro bubbles functioning as a reflection source. Because of properties of the bubbles as a delicate base material, even in case of ultrasonic irradiation on a regular diagnostic level, the bubbles are destroyed due to a mechanical action of this ultrasonic irradiation, and signal intensity from a scan plane is thereby reduced. Therefore, in order to observe a dynamic state of a plenishment in real time, it is necessary to perform a relative reduction in destruction of the bubbles due to scanning, e.g., imaging by ultrasonic transmission of a low sound pressure. Such imaging based on ultrasonic transmission of a low sound pressure also decreases a signal/noise ratio (an S/N ratio), and hence various kinds of signal processing methods that compensate such a decrease have been designed.

Furthermore, the characteristics that the contrast medium bubbles are destroyed are exploited to design the following technique. That is, (a) a moving state of bubbles filling a scan cross section under irradiation of a low sound pressure is observed, (b) the irradiation sound pressure is changed to a high sound pressure to destroy the bubbles in the cross section (which is an irradiation volume in a narrow sense), and (c) a state of the bubbles flowing into the cross section is again observed. This technique is called a flash-replenishment (which will be referred to as an FR method hereinafter) (see, e.g., JP-A 1997 324 772 (KOKAI)). Furthermore, in recent years, an apparatus that enables three-dimensional scanning in real time has appeared, and the above-explained technique has been three-dimensionally applied.

Meanwhile, a so-called "next-generation contrast medium" that emits a harmonic signal to enable long-time visualization without being destroyed even if ultrasonic waves with a low sound pressure are transmitted has recently gone on the market. Sonazoid (a registered trademark) as this next-generation contrast medium is micro bubbles that contain a perfluorobutane gas and have phospholipid serving as a shell, and has properties that it is taken into Kupffer cells in the liver with time. In Sonazoid, the number of micro bubbles which are substantially taken in is very large. Moreover, when the bubbles having a high density are destroyed, since a large amount of ultrasonic pulse energy is consumed by the bubbles near the destroyed counterparts, and hence it takes time to destroy bubbles placed at a deep part.

For example, in case of destroying bubbles in a scan cross section, destroying bubbles corresponding to, e.g., approximately five frames is sufficient when using any other contrast medium, but destroying bubbles corresponding to approximately 30 frames may be insufficient when using Sonazoid. As a result, not only destroying bubbles takes time, but also remaining undestroyed bubbles may be also mixed when a visualizing method of tracing bubbles like a maximum intensity projection (which will be referred to as an MIP method thereinafter) method is used for a luminance of a state of reflow after the bubbles are cleaned away, thereby obstructing a diagnosis.

As explained above, when a contrast medium in which the number of bubbles is very large and the bubbles are hardly destroyed like Sonazoid is used, there occurs a problem that destroying all the bubbles takes time and a diagnosis is performed for a long time. Additionally, in case of using the visualizing method of tracing bubbles like the MIP method, when remaining undestroyed bubbles are present, these bubbles are visualized, and an accurate diagnostic result may not be obtained.

US 2007/167799 A1 relates to an ultrasonic diagnostic apparatus according to the preamble of claim 1.

In view of the above-explained circumstances, it is an object of the present invention to provide an ultrasonic diagnostic apparatus and a program for controlling such apparatus that can reliably eliminate contrast medium bubbles in a short time.

This is achieved by the ultrasonic diagnostic apparatus of claim 1 and the program for controlling an ultrasonic diagnostic apparatus of claim 4.

Further advantageous embodiments of the invention are specified in the dependent claims.

The invention can be more fully understood from the following detailed description when taken in conjunction with the accompanying drawings, in which:
FIG. 1 is a view showing an example of a block structure of an ultrasonic diagnostic apparatus according to a first embodiment;
FIG. 2 shows an example of a basic scan sequence;
FIG. 3 shows an example of a scan sequence that changes a transmission focal position;
FIG. 4 shows an example of an ultrasonic image representing a state before bubble destruction in a phantom experiment;
FIG. 5 shows an example of an ultrasonic image representing a state after bubbles are destroyed by a technique of Example 1 in the phantom experiment;
FIG. 6 shows an example of an ultrasonic image representing a state after bubbles are destroyed by a conventional technique in the phantom experiment;
FIG. 7 shows an example of a scan sequence that changes a sound pressure; and
FIG. 8 shows an example of a technique that obtains a transmission focal position from a luminance distribution of a contrast image.

An embodiment according to the present invention will now be explained hereinafter with reference to the drawings. It is to be noted that like numerals denote constituent elements having substantially the same functions and structures, and a tautological explanation will be given only when needed.

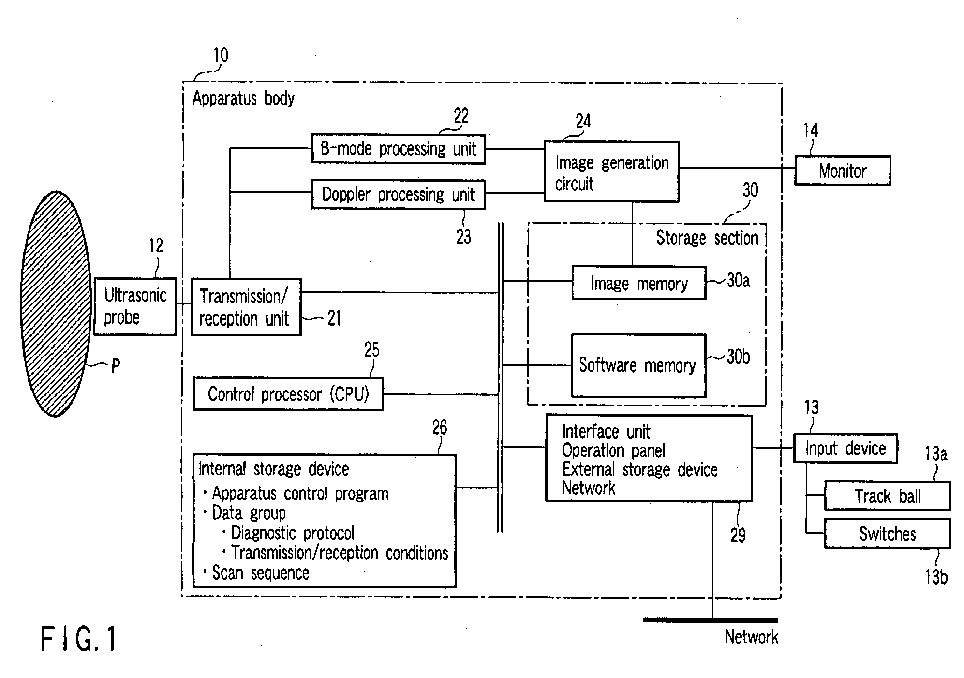

FIG. 1 is a block diagram showing an ultrasonic diagnostic apparatus according to this embodiment. As shown in the drawing, this ultrasonic diagnostic apparatus includes an ultrasonic probe 12, an input device 13, a monitor 14, a transmission/reception unit 21, a B-mode processing unit 22, a Doppler processing unit 23, an image generation circuit 24, a control processor (CPU) 25, an internal storage device 26, an interface unit 29, and a storage unit 30 having an image memory 30a and a software memory 30b. The transmission/reception unit 21 or the like included in an apparatus body 10 may be constituted of hardware such as an integrated circuit, but it may be a software program formed as a module in terms of software. A function of each constituent element will now be explained.

The ultrasonic probe 12 generates ultrasonic waves based on a driving signal from the transmission/reception unit 21, and has a plurality of piezoelectric transducers that convert a reflected wave from a subject into an electric signal, a matching layer provided to each piezoelectric transducer, a backing material that prevents ultrasonic waves from being propagated to parts behind the piezoelectric transducers, and others. When ultrasonic waves are transmitted from the ultrasonic probe 12 to a subject P, the transmitted ultrasonic waves are sequentially reflected on an acoustic impedance discontinuous surface of a body tissue and received as an echo signal by the ultrasonic probe 12. An amplitude of this echo signal is dependent on a difference between acoustic impedances on the discontinuous surface where reflection occurs. Further, in case of an echo when reflected on a surface of, e.g., a moving blood flow or a heart wall, a transmitted ultrasonic pulse undergoes frequency shift in dependent on a velocity component of a movable body in an ultrasonic transmitting direction based on a Doppler effect.

The input device 13 is connected with the apparatus body 10 and has a track ball 13a, various kinds of switches 13b, a button, a mouse, a keyboard, and others to take various instructions, conditions, a region-of-interest (ROI) setting instruction, various image quality condition setting instructions, and others from an operator into the apparatus body 10.

The monitor 14 displays intravital morphological information or blood flow information as an image based on a video signal output from the image generation circuit 24.

The transmission/reception unit 21 has a trigger generation circuit, a delay circuit, a pulsar circuit, and others (not shown). The pulsar circuit repeatedly generates a rate pulse to form transmission ultrasonic waves with a predetermined rate frequency fr Hz (period; 1/fr sec). Further, the delay circuit gives each rate pulse a delay time required to focus an ultrasonic wave into a beam-like shape and determine transmission directivity for each channel. The trigger generation circuit applies a driving pulse to the probe 12 at a timing based on this rate pulse.

It is to be noted that the transmission/reception unit 21 has a function enabling instantaneously changing, e.g., transmission focusing, a sound pressure, a transmission frequency, a transmission driving voltage, a transmission region, or a transmission pulse rate in order to execute later-explained scanning of this embodiment in accordance with an instruction from the control processor 25. In particular, the transmission driving voltage is changed by a linear amplifier type transmission circuit that can instantaneously change a value of this voltage or a mechanism that electrically switches a plurality of power supply units.

Furthermore, the transmission/reception unit 21 has an amplifier circuit, an A/D converter, an adder, and others (not shown). The amplifier circuit amplifies an echo signal received through the probe 12 for each channel. The A/D converter gives the amplified echo signal a delay time required to determine reception directivity, and then performs addition processing in the adder. Based on this addition, a reflection component in the echo signal from a direction conforming to the reception directivity is emphasized, and a comprehensive beam for ultrasonic transmission/reception is formed based on the reception directivity and the transmission directivity.

The B-mode processing unit 22 receives the echo signal from the transmission/reception unit 21 and performs, e.g., logarithmic amplification or envelope detection processing to generate data representing a signal intensity by a luminance. This data is transmitted to the image generation circuit 24 and displayed as a B-mode image representing an intensity of a reflected wave by a luminance in the monitor 14. It is to be noted that the signal processed here may be a nonlinear signal component extracted from the echo signal. The nonlinear signal component is also generated by a reflected wave from a living body, and it is useful for efficient visualization of a reflected wave from bubbles.

The Doppler processing unit 23 performs frequency analysis with respect to velocity information from the echo signal received from the transmission/reception unit 21, and extracts components of a blood flow, a tissue, a contrast medium echo based on the Doppler effect to obtain blood flow information such as an average velocity, dispersion, a power, and others at many points. The obtained blood flow information is transmitted to the image generation circuit 24 to be displayed as an average velocity image, a dispersion image, a power image, and an image of a combination of these elements in color in the monitor 14. When a tissue signal that appears as a low flow velocity is removed by filtering, a signal from bubbles alone can be visualized, and this image is utilized to obtain a transmission parameter concerning later-explained second transmission.

The image generation circuit 24 converts a scan line signal string of ultrasonic scan into a scan line signal string having a general video format as typified by, e.g., a television, thereby generating an ultrasonic diagnostic image as a display image. The image generation circuit 24 has a storage memory storing image data mounted thereon so that an operator can bring up an image recorded during an examination after diagnosis, for example. Thus, a tomogram showing a subject tissue shape is displayed.

The control processor (CPU) 25 functions as an information processor (computer) and controls operations of this ultrasonic diagnostic apparatus body. The control processor 25 reads out a control program required to execute later-explained ultrasonic transmission/reception, image generation, display, and others from the internal storage device 26 to be spread on the software storing unit 30b, and carries out an arithmetic operation or control concerning various kinds of processing.

The internal storage device 26 stores a control program required to execute later-explained scan sequence, image generation, and display processing, diagnostic information (a patient ID, a remark of a doctor, and others), a diagnostic protocol, transmission/reception conditions, and any other data groups. In particular, the internal storage device 26 stores a control program required to execute second ultrasonic transmission based on a scan sequence. Furthermore, it is also used to store images in the image memory 30a as required. Data in the internal storage device 26 can be transferred to an external peripheral device through the interface unit 29.

The interface unit 29 is an interface concerning the input device 13, a network, and a new external storage device (not shown). Data such as an ultrasonic image, an analysis result, and others obtained by this apparatus can be transferred to other devices through the network.

The image memory 30a is formed of a storage memory that stores image data received from the image generation circuit 24. An operator can bring up this image data after, e.g., a diagnosis, and this image data can be reproduced as a still image or as an moving image when a plurality of images are used. Further, the image memory 30a also stores a signal (which is called a radio frequency (RF) signal) immediately after output from the transmission/reception unit 21, an image luminance signal after passed through the transmission/reception unit 21, any other raw data, image data acquired through the network, and others as required.

### (Basic Scan Sequence)

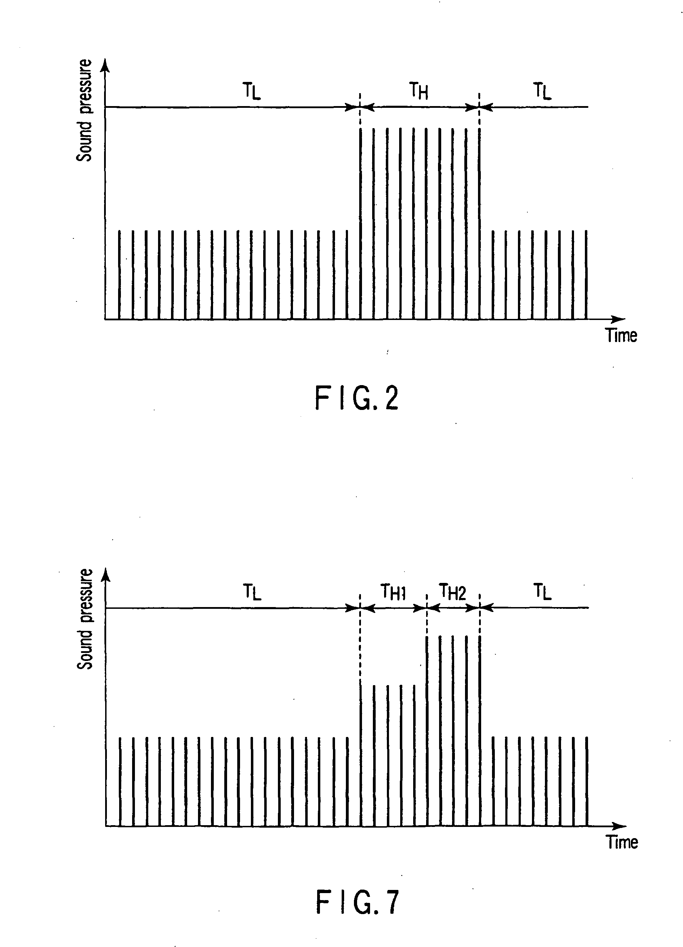

A basic scan sequence executed by this ultrasonic diagnostic apparatus will now be explained with reference to FIG. 2. This scan sequence alternately executes two types of transmission using different sound pressures, i.e., high sound pressure transmission for destruction of contrast medium bubbles and a low sound pressure transmission for acquisition of a diagnostic image while preventing destruction of bubbles as much as possible in a contrast echo utilizing a contrast medium. It is to be noted that a contrast medium preferable to be used in imaging according to this sequence is a so-called "next-generation contrast medium" which emits a harmonic signal without being destroyed even if ultrasonic waves of a low sound pressure are transmitted and enables visualization for a long time.

FIG. 2 is a view for explaining the basic scan sequence, in which an abscissa represents a time and an ordinate represents a degree of a mechanical action with respect to bubbles based on ultrasonic transmission. Furthermore, each line represents ultrasonic scan concerning one frame (or one volume (which will be likewise applied to the following)), and a length of each line represents mechanical action strength of a transmission sound pressure of each frame. That is, each line represents ultrasonic scan for one frame based on transmission conditions determined in such a manner that a transmission frequency is reduced or a transmission driving sound pressure is increased or both are achieved as a length of each line in the vertical direction becomes long (large). Scan based on low sound pressure irradiation indicated by a period T_{L} in FIG. 2 will be referred to as first ultrasonic transmission, and scan based on high sound pressure irradiation indicated by a period T_{H} (corresponding to 10 frames in the drawing) will be referred to as second transmission. Moreover, a tomographic image obtained by the low sound pressure irradiation after this second transmission is called a replenishment image. Additionally, a tomographic image obtained by frame scan immediately before changing to high sound pressure irradiation in scan based on low sound pressure irradiation is called a pre-flash image. It is to be noted that one frame is formed of a plurality of scan lines, and hence one line symbolically represents several hundred times of transmission/reception concerning the plurality of scan lines.

In the low sound pressure irradiation, since destruction of the contrast medium bubbles is small, the number of bubbles flowing into a scan cross section is gradually increased, and an equilibrium state is reached in observation for a long time. Upon changing to the high sound pressure transmission, the bubbles in the cross section are precipitously destroyed, and the bubbles are substantially completely eliminated by irradiation for the number of times corresponding to at least one frame, or preferably approximately 30 frames. Further, when the transmission is again changed to the low sound pressure transmission and the replenishment images are observed, a state of replenishment can be observed. When this procedure is repeatedly carried out, a replenishment phenomenon can be repeatedly observed by using the pre-flash images.

Meanwhile, in the ultrasonic diagnostic apparatus according to this embodiment, a transmission parameter concerning the second transmission is changed in accordance with time. Specific contents of the second ultrasonic transmission processing will now be explained based on Examples.

### (Example 1)

In Example 1, a position of transmission focus (a focal point) is changed in accordance with time in the second ultrasonic transmission processing.

Usually, when destroying all ultrasonic contrast medium bubbles in a scan volume, it is general to focus on the deepest position in the scan volume to execute high sound pressure transmission. That is because increasing a sound pressure at a deep part at which destroying bubbles is most difficult is important in order to attenuate ultrasonic waves.

When an amount of bubbles is very large, ultrasonic pulse energy is lost when bubbles in a close range are destroyed, and it is greatly attenuated at a non-focused shallow part. Therefore, bubbles are gradually destroyed from a close range to eventually destroy all the bubbles in the scan volume, thereby requiring a long time.

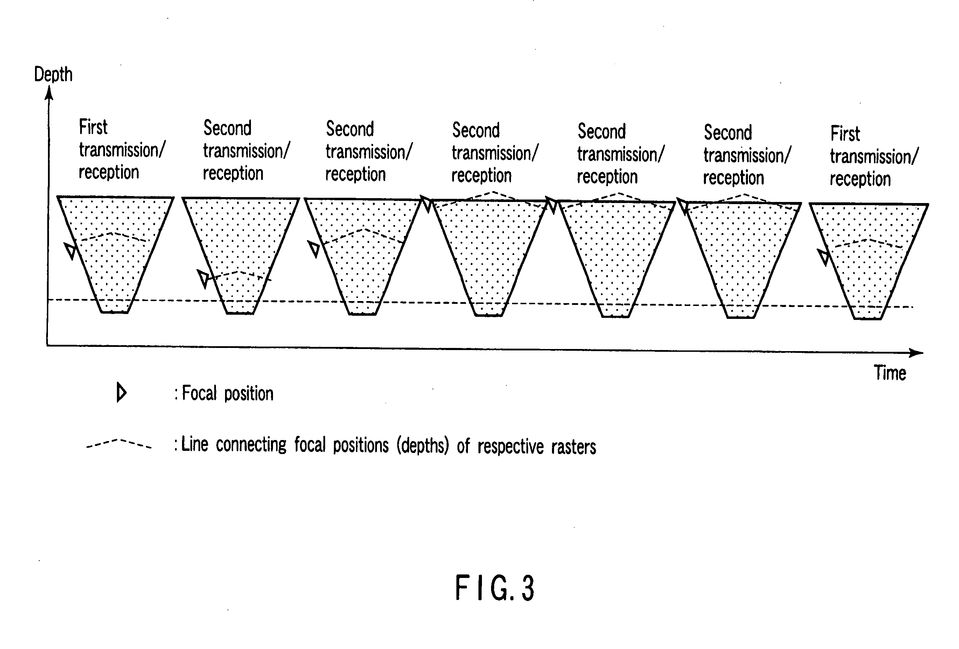

Thus, the control processor 25 controls the transmission/reception unit 21 to focus on a shallow part at the beginning and then focus on deeper positions in the second transmission. FIG. 3 shows an example of this control where a depth of focusing (an ordinate) is changed in accordance with time (an abscissa). Since it can be considered that an efficiency for elimination of bubbles is increased when a shallow part is focused at the beginning to destroy bubbles in a close range and then destroy bubbles at a deep part because destruction of the bubbles starts from the close range, a time required for the second transmission can be reduced.

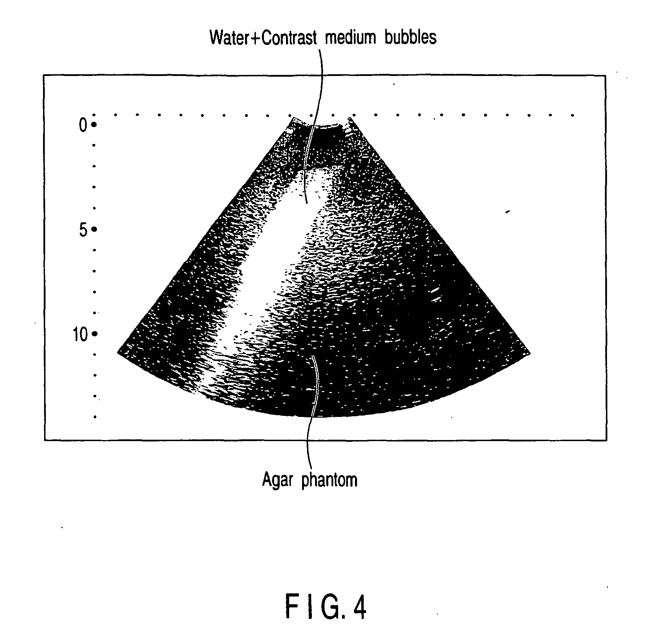

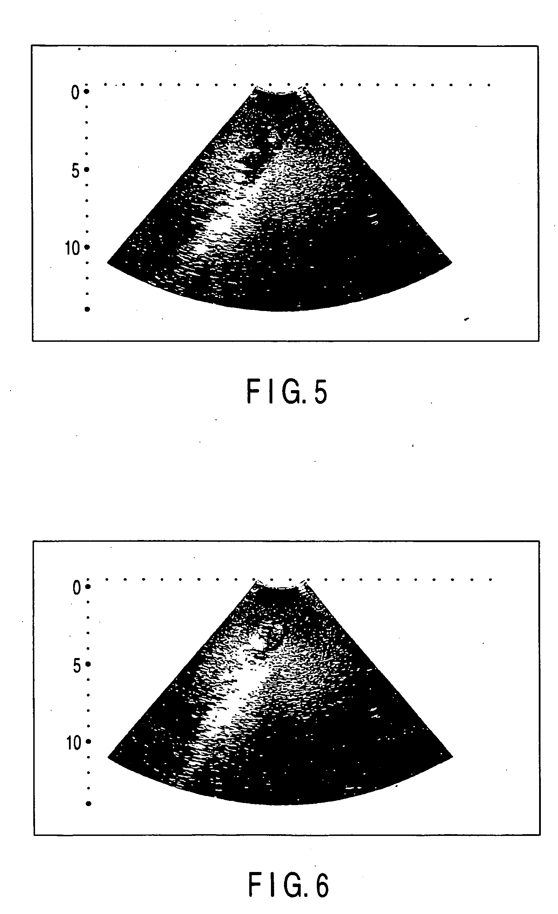

A result of an experiment using a phantom will now be explained. FIG. 4 shows a state immediately before destroying bubbles based on observation in the first ultrasonic transmission. A white part represents contrast medium bubbles. FIG. 5 shows a state after changing a focal position to four cm, seven cm, nine cm, and 12 cm in the second ultrasonic transmission based on observation in the first ultrasonic transmission. FIG. 6 shows a state after a focal position is fixed to 14 cm and the second ultrasonic transmission is performed with the same irradiation time as that in the example of FIG. 5 like the conventional technique. Comparing FIGS. 5 and 6, it can be understood that more bubbles are destroyed by focusing on a shallow part at the beginning to start destruction of bubbles in a close range like the technique in this Example 1 as compared with an example of focusing on a deep part from the beginning to perform ultrasonic transmission like the conventional technique. Therefore, according to this technique, the bubbles can be swept away in a short time.

### (Example 2)

According to Example 2, which does not form part of this invention, a sound pressure is changed with time in the second ultrasonic transmission processing.

For example, when a sound pressure is increased to a maximum level that can be realized by the apparatus when the second transmission is performed with respect to the next-generation ultrasonic contrast medium, a large quantity of bubbles can be destroyed whereas a loss of transmission energy at the time of destruction of bubbles is also large. Therefore, when a sound pressure is not increased to the maximum level and the bubbles are destroyed with a sound pressure enabling properly destroying the bubbles, the loss of energy can be suppressed and the bubbles can be efficiently destroyed.

Thus, as shown in FIG. 7, the control processor 25 controls the transmission/reception unit 21 in such a manner that a sound pressure is reduced at the beginning (period T_{H1} in FIG. 7) in the second transmission and then the sound pressure is increased to a maximum level (period T_{H2} in FIG. 7). FIG. 7 shows an example where the sound pressure is changed on two stages, but it may be gradually changed. Furthermore, lengths of the period T_{H1} and the period T_{H2} can be arbitrarily changed.

In particular, when transmission with the maximum sound pressure is carried out with respect to bubbles in a close range, the sound pressure in the close range becomes very high. That is, a sequence that the sound pressure is reduced when destroying bubbles in the close range and the sound pressure is increased when destroying bubbles in a deep part can be also considered.

### (Example 3)

According to Example 3 which does not from part of this invention, a transmission frequency is changed with time in the second ultrasonic transmission processing.

For example, since large transmission pulse energy is required to destroy large bubbles, a transmission frequency must be reduced. On the other hand, many small bubbles can be destroyed at a time with a high transmission frequency. Thus, the control processor 25 controls the transmission/reception unit 21 in such a manner that the transmission frequency is increased at the beginning and then it is reduced in the second transmission. When such control is executed, since small bubbles can be swept away at the beginning, the loss of the transmission pulse energy due to destruction of the small bubbles can be suppressed, and large bubbles which are hard to be destroyed can be efficiently destroyed.

### (Example 4)

According to Example 4, a luminance distribution is calculated in a depth direction from a contrast image, and transmission parameters are changed based on this luminance distribution.

For example, when a region having no bubble is focused in the second transmission, an efficiency of destroying bubbles is lowered as compared with an example where a deep part is focused. Moreover, when a scan region is changed during high sound pressure irradiation (e.g., when the probe is moved), it can be considered that the loss of the transmission pulse energy again occurs due to destruction of bubbles in a shallow part.

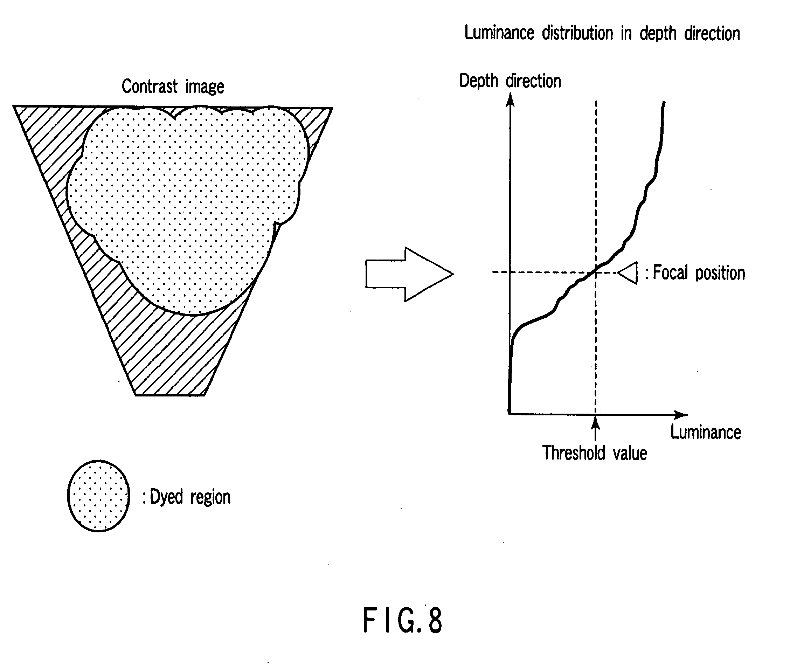

To solve this problem, FIG. 8 shows an example of a technique of obtaining a transmission focal position from a luminance distribution of a contrast image. The control processor 25 obtains a luminance distribution in a dye depth direction from an image acquired from the first transmission/reception immediately before performing the second transmission/reception, and sets a focal position at a depth where a luminance exceeds a predetermined threshold value to start the second transmission by the transmission/reception unit 21.

It is to be noted that a sound pressure value may be obtained from not only the focal position but also the luminance distribution. Additionally, the Doppler processing unit 23 may visualize a signal from bubbles alone during the second transmission to obtain a focal position, a sound pressure, a transmission frequency, and others with the above-explained algorithm from the image acquired from the bubbles alone. With the above-explained operation, suitable transmission parameters can be automatically obtained in accordance with a bubble presence situation.

According to the above-explained structure, the following effects can be obtained.

According to this ultrasonic diagnostic apparatus, changing transmission parameters concerning the second transmission for bubble destruction with time enables efficiently eliminating the bubbles. As explained above, for example, when a transmission focal position is changed from a shallow part to a deep part with time, the bubbles can be efficiently destroyed from the close range to the deep part, a time required for destruction can be thereby reduced, and the bubbles in the entire scan region can be assuredly destroyed.

Further, according to this ultrasonic diagnostic apparatus, a luminance distribution is obtained in a depth direction from a contrast image acquired by the first ultrasonic transmission performed immediately before the second transmission or a contrast image acquired by the second transmission, and transmission parameters are changed in accordance with this luminance distribution. When such an operation is carried out, since the transmission parameter narnely transmission focusing, can be obtained in accordance with a bubble presence situation, the bubbles can be more assuredly destroyed in a shorter time.

It is to be noted that the present invention is not restricted to the foregoing embodiment as it is, and its constituent elements can be modified and carried out without departing from the scope of the invention as defined by the appended claims. Specific modifications are as follows, for example.
(1) Each function concerning this embodiment can be also realized by installing a program executing the processing in a computer such as a work station and spreading the installed program on a memory. At this time, the program that allows the computer to execute the technique can be stored in a recording medium such as a magnetic disk (e.g., a floppy (a registered trademark) disk or a hard disk), an optical disk (e.g., a CD-ROM or a DVD), or a semiconductor memory to be distributed.
(2) In the foregoing embodiment, the transmission/reception unit 21 may execute the second ultrasonic transmission in accordance with a B mode or may execute the same in accordance with a Doppler mode.
(3) In the foregoing embodiment, the example where the transmission region of the first ultrasonic transmission is equal to the transmission region of the second ultrasonic transmission has been explained, but the present invention is not restricted thereto, and the transmission region of the second ultrasonic transmission may be a part of the transmission region of the first ultrasonic transmission.
(4) Although the ultrasonic diagnostic apparatus that generates a two-dimensional image has been explained in the foregoing embodiment, the present invention can be likewise applied to an ultrasonic diagnostic apparatus that generates a three-dimensional image (volume data).

## Claims

1. An ultrasonic diagnostic apparatus comprising:
an ultrasonic probe (12) adapted to transmit an ultrasonic wave to a subject dosed with contrast medium bubbles, to receive a reflected wave, and to generate an echo signal;
a transmission unit (21) adapted to execute through the ultrasonic probe (12) first ultrasonic transmission using a first sound pressure as a sound pressure that does not substantially destroy the contrast medium bubbles and is required to obtain an ultrasonic image of the subject and second ultrasonic transmission using a second sound pressure as a sound pressure that is required to destroy the contrast medium bubbles and higher than the first sound pressure based on a transmission parameter in accordance with each frame or each volume;
an image generation unit (24) adapted to use the echo signal obtained by the first ultrasonic transmission to generate the ultrasonic image; and
a control unit (25) adapted to change the transmission parameter concerning the second ultrasonic transmission in accordance with time,
**characterized in that**
the transmission parameter is a focal point, and
the control unit (25) is adapted to move the focal point of the second ultrasonic transmission from a shallow part to a deep part.

2. The apparatus according to claim 1, **characterized in that** the control unit (25) is adapted to obtain a luminance distribution in a depth direction from the ultrasonic image generated by the first ultrasonic transmission before performing the second ultrasonic transmission, and to change the focal point of the second ultrasonic transmission in accordance with the luminance distribution.

3. The apparatus according to claim 1, **characterized in that** the image generation unit (24) is further adapted to use the echo signal obtained by the second ultrasonic transmission to generate an ultrasonic image of a contrast part of the subject, and
the control unit (25) is adapted to obtain a luminance distribution in a depth direction from the ultrasonic image of the contrast part, and to change the focal point of the second ultrasonic transmission in accordance with the luminance distribution.

4. A program for controlling an ultrasonic diagnostic apparatus including an ultrasonic probe adapted to transmit an ultrasonic wave to a subject dosed with contrast medium bubbles, to receive a reflected wave, and to generate an echo signal, the program allowing a computer to execute:
a function of executing first ultrasonic transmission using a first sound pressure as sound pressure that does not substantially destroy the contrast medium bubbles and is required to obtain an ultrasonic image of the subject and second ultrasonic transmission using a second sound pressure as a sound pressure that is required to destroy the contrast medium bubbles and higher than the first sound pressure based on a transmission parameter in accordance with each frame or each volume; and
a function of changing the transmission parameter concerning the second ultrasonic transmission in accordance with time,
**characterized in that**
the transmission parameter is a focal point, and
the focal point of the second ultrasonic transmission is moved from a shallow part to a deep part of the subject.

5. The program according to claims 4, **characterized in that** a luminance distribution in a depth direction is obtained from the ultrasonic image generated by the first ultrasonic transmission before performing the second ultrasonic transmission, and the focal point of the second ultrasonic transmission is changed in accordance with the luminance distribution.

6. The program according to claim 4, **characterized in that** an ultrasonic image of a contrast part of the subject is generated by using the echo signal obtained by the second ultrasonic transmission, and
a luminance distribution in a depth direction is obtained from the ultrasonic image of the contrast part, and the focal point of the second ultrasonic transmission is changed in accordance with the luminance distribution.

## Patentansprüche

1. Ultraschalldiagnosevorrichtung mit:
einer Ultraschallsonde (12), die zum Übertragen einer Ultraschallwelle zu einem Subjekt, dem Kontrastmittelblasen verabreicht wurden, zum Empfangen einer reflektierten Welle und zum Erzeugen eines Echosignals angepasst ist,
einer Übertragungseinheit (21), die zum Ausführen einer ersten Ultraschallübertragung unter Verwendung eines ersten Schalldrucks als einen Schalldruck, der die Kontrastmittelblasen nicht wesentlich zerstört und zum Erhalten eines Ultraschallbilds des Subjekts erforderlich ist,
und einer zweiten Ultraschallübertragung unter Verwendung eines zweiten Schalldrucks als einen Schalldruck, der zum Zerstören der Kontrastmittelblasen erforderlich und höher als der erste Schalldruck ist, durch die Ultraschallsonde (12) basierend auf einem Übertragungsparameter gemäß jedem Rahmen oder jedem Volumen angepasst ist,
einer Bilderzeugungseinheit (24), die zum Verwenden des Echosignals, das durch die erste Ultraschallübertragung erhalten wird, zur Erzeugung des Ultraschallbilds angepasst ist, und
einer Steuerungseinheit (25), die zum Ändern des Übertragungsparameters, der die zweite Ultraschallübertragung betrifft, mit der Zeit angepasst ist,
**dadurch gekennzeichnet, dass**
der Übertragungsparameter ein Brennpunkt ist, und
die Steuerungseinheit (25) zum Bewegen des Brennpunkts der zweiten Ultraschallübertragung von einem Teil niedriger Tiefe zu einem tiefen Teil angepasst ist.

2. Vorrichtung nach Anspruch 1, **dadurch gekennzeichnet, dass** die Steuerungseinheit (25) zum Erhalten einer Leuchtdichteverteilung in einer Tiefenrichtung aus dem Ultraschallbild, das durch die erste Ultraschallübertragung erzeugt wird, vor einem Durchführen der zweiten Ultraschallübertragung und zum Ändern des Brennpunkts der zweiten Ultraschallübertragung gemäß der Leuchtdichteverteilung angepasst ist.

3. Vorrichtung nach Anspruch 1, **dadurch gekennzeichnet, dass** die Bilderzeugungseinheit (24) ferner zum Verwenden des Echosignals, das durch die zweite Ultraschallübertragung erhalten wird, zur Erzeugung eines Ultraschallbilds eines Kontrastteils des Subjekts angepasst ist, und
die Steuerungseinheit (25) zum Erhalten einer Leuchtdichteverteilung in einer Tiefenrichtung aus dem Ultraschallbild des Kontrastteils und zum Ändern des Brennpunkts der zweiten Ultraschallübertragung gemäß der Leuchtdichteverteilung angepasst ist.

4. Programm zum Steuern einer Ultraschalldiagnosevorrichtung, die eine Ultraschallsonde, die zum Übertragen einer Ultraschallwelle zu einem Subjekt, dem Kontrastmittelblasen verabreicht wurden, zum Empfangen einer reflektierten Welle und zum Erzeugen eines Echosignals angepasst ist, aufweist, wobei das Programm einem Rechner erlaubt, folgende Funktionen auszuführen:
eine Funktion zum Ausführen einer ersten Ultraschallübertragung unter Verwendung eines ersten Schalldrucks als einen Schalldruck, der die Kontrastmittelblasen nicht wesentlich zerstört und zum Erhalten eines Ultraschallbilds des Subjekts erforderlich ist, und einer zweiten Ultraschallübertragung unter Verwendung eines zweiten Schalldrucks als einen Schalldruck, der zum Zerstören der Kontrastmittelblasen erforderlich und höher als der erste Schalldruck ist, basierend auf einem Übertragungsparameter gemäß jedem Rahmen oder jedem Volumen, und eine Funktion zum Ändern des Übertragungsparameters, der die zweite Ultraschallübertragung betrifft, mit der Zeit,
**dadurch gekennzeichnet, dass**
der Übertragungsparameter ein Brennpunkt ist, und
der Brennpunkt der zweiten Ultraschallübertragung von einem Teil niedriger Tiefe zu einem tiefen Teil des Subjekts bewegt wird.

5. Programm nach Anspruch 4, **dadurch gekennzeichnet, dass** eine Leuchtdichteverteilung in einer Tiefenrichtung aus dem Ultraschallbild, das durch die erste Ultraschallübertragung erzeugt wird, vor dem Durchführen der zweiten Ultraschallübertragung erhalten wird und der Brennpunkt der zweiten Ultraschallübertragung gemäß der Leuchtdichteverteilung geändert wird.

6. Programm nach Anspruch 4, **dadurch gekennzeichnet, dass** ein Ultraschallbild eines Kontrastteils des Subjekts unter Verwendung des Echosignals, das durch die zweite Ultraschallübertragung erhalten wird, erzeugt wird, und
eine Leuchtdichteverteilung in einer Tiefenrichtung auf dem Ultraschallbild des Kontrastteils erhalten wird und der Brennpunkt der zweiten Ultraschallübertragung gemäß der Leuchtdichteverteilung geändert wird.

## Revendications

1. Appareil de diagnostic à ultrasons comprenant :
une sonde à ultrasons (12) adaptée pour transmettre une onde ultrasonique à un patient ayant une dose de bulles de milieu de contraste, pour recevoir une onde réfléchie et générer un signal d'écho ;
une unité de transmission (21) adaptée pour exécuter via la sonde à ultrasons (12), une première transmission ultrasonique en utilisant une première pression sonore comme pression sonore qui ne détruit sensiblement pas les bulles de milieu de contraste et qui est requise pour obtenir une image ultrasonique du patient et une seconde transmission ultrasonique en utilisant une seconde pression sonore comme pression sonore qui est requise pour détruire les bulles de milieu de contraste et supérieure à la première pression sonore sur la base d'un paramètre de transmission en fonction de chaque trame ou de chaque volume ;
une unité de génération d'image (24) adaptée pour utiliser le signal d'écho obtenu par la première transmission ultrasonique pour générer l'image ultrasonique ; et
une unité de commande (25) adaptée pour modifier le paramètre de transmission concernant la seconde transmission ultrasonique en fonction du temps,
**caractérisé en ce que**
le paramètre de transmission est un point focal et
l'unité de commande (25) est adaptée pour déplacer le point focal de la seconde transmission ultrasonique d'une partie peu profonde à une partie profonde.

2. Appareil selon la revendication 1, **caractérisé en ce que** l'unité de commande (25) est adaptée pour obtenir une répartition de la luminance dans une direction en profondeur à partir de l'image ultrasonique générée par la première transmission ultrasonique avant d'effectuer la seconde transmission ultrasonique et pour modifier le point focal de la seconde transmission ultrasonique en fonction de la répartition de la luminance.

3. Appareil selon la revendication 1, **caractérisé en ce que** l'unité de génération d'image (24) est également adaptée pour utiliser le signal d'écho obtenu par la seconde transmission ultrasonique pour générer une image ultrasonique d'une partie de contraste du patient, et
l'unité de commande (25) est adaptée pour obtenir une répartition de la luminance dans une direction en profondeur à partir de l'image ultrasonique de la partie de contraste et pour modifier le point focal de la seconde transmission ultrasonique en fonction de la répartition de la luminance.

4. Programme de commande d'un appareil de diagnostic à ultrasons comprenant une sonde à ultrasons adaptée pour transmettre une onde ultrasonique à un patient ayant une dose de bulles de milieu de contraste, pour recevoir une onde réfléchie et générer un signal d'écho, le programme permettant à un ordinateur d'exécuter :
une fonction d'exécution d'une première transmission ultrasonique en utilisant une première pression sonore comme pression sonore qui ne détruit sensiblement pas les bulles de milieu de contraste et qui est requise pour obtenir une image ultrasonique du patient et une seconde transmission ultrasonique en utilisant une seconde pression sonore comme pression sonore qui est requise pour détruire les bulles de milieu de contraste et supérieure à la première pression sonore sur la base d'un paramètre de transmission en fonction de chaque trame ou de chaque volume ; et
une fonction de modification du paramètre de transmission concernant la seconde transmission ultrasonique en fonction du temps,
**caractérisé en ce que**
le paramètre de transmission est un point focal et
le point focal de la seconde transmission ultrasonique est déplacé d'une partie peu profonde à une partie profonde du sujet.

5. Programme selon la revendication 4, **caractérisé en ce qu'**une répartition de la luminance dans une direction en profondeur est obtenue à partir de l'image ultrasonique générée par la première transmission ultrasonique avant d'effectuer la seconde transmission ultrasonique et le point focal de la seconde transmission ultrasonique est modifié en fonction de la répartition de la luminance.

6. Programme selon la revendication 4, **caractérisé en ce qu'**une une image ultrasonique d'une partie de contraste du patient est générée en utilisant le signal d'écho obtenu par la seconde transmission ultrasonique, et
une répartition de la luminance dans une direction en profondeur est obtenue à partir de l'image ultrasonique de la partie de contraste et le point focal de la seconde transmission ultrasonique est modifié en fonction de la répartition de la luminance.
